# EUROPÄISCHE PATENTANMELDUNG

(11) **EP 0 687 444 A2**
(43) Veröffentlichungstag der Anmeldung: **20.12.1995**
(21) Anmeldenummer: 95107540.7
(22) Anmeldetag: 18.05.1995
(51) Int. Cl.: A61B 6/04, A61B 6/08

(54) **Vorrichtung zur Positionierung und Markierung eines Patienten an Diagnosegeräten, z.B. vor und nach der Durchleuchtung in einem Computertomographen**

(30) Priorität: 17.06.1994 DE 4421316
(71) Anmelder: LAP GmbH Laser Applikationen, D-21337 Lüneburg (DE)
(72) Erfinder: Röckseisen, Armin, Dr., D-21379 Scharnebeck (DE)
(74) Vertreter: Dipl.-Ing. H. Hauck, Dipl.-Ing. E. Graalfs, Dipl.-Ing. W. Wehnert, Dr.-Ing. W. Döring, Dr.-Ing. N. Siemons

(57) **Zusammenfassung**

Vorrichtung zur Positionierung und Markierung eines Patienten an Diagnosegeräten, z.B. vor und nach der Durchleuchtung in einem Computertomographen, mit Linienlasern, die im Raum in aufeinander senkrecht stehenden Ebenen Linien projizieren zur Ausrichtung auf ein zu bestrahlendes Gebiet des Patientenkörpers und Markierung desselben, wobei ein erster Linienlaser (22) auf einem Lagerkörper (10) angebracht ist, der um eine Achse (12) gelagert ist, die annähernd mit der zentralen Achse des Diagnosegerätes, z.B. Computertomographen (30) ausrichtbar ist, wobei der Lichtstrahl des außermittig angeordneten Linienlasers (22) in einer durch die Z-Achse hindurchgehenden Ebene liegt, die auf einer zwischen Diagnosegerät, z.B. Computertomograph (30) und Lagerkörper (10) angeordneten Liege (32) senkrecht steht, wenn der Lagerkörper (10) in Nullposition ist, daß der Lagerkörper durch einen numerisch gesteuerten Antrieb antreibbar ist und eine Steuervorrichtung vorgesehen ist zur Verstellung des Lagerkörpers nach Maßgabe von Steuerdaten, die aus den durch das Diagnosegerät, z.B. Computertomograph ermittelten Koordinaten eines Tumors ermittelt werden.

## Beschreibung

Die Erfindung bezieht sich auf eine Vorrichtung zur Positionierung und Markierung eines Patienten an Diagosegeräten, z.B. vor und nach der Durchleuchtung in einem Computertomographen nach dem Oberbegriff des Patentanspruchs 1.

In der Strahlentherapie ist es notwendig, den Strahl der Bestrahlungsquelle präzise auf das zu bestrahlende Gebiet eines Patientenkörpers auszurichten, und zwar reproduzierbar. Zur Verringerung der Belastung nicht zu therapierender Bereiche wird die Strahlungsquelle um ein sogenanntes Isozentrum geschwenkt, so daß im Zentrum des zu bestrahlenden Gebietes stets eine gleiche Dosisleistung erzielt wird, im benachbarten nicht zu behandelnden Gebiet hingegen eine deutlich verringerte Belastung stattfindet. Die Positionierung eines Patienten im Hinblick auf das Bestrahlungsgerät ist so auszurichten, daß das Zentrum des zu bestrahlenden Gebietes mit dem Isozentrum des Bestrahlungsgerätes zusammenfällt. Die Lage des zu bestrahlenden Gebietes kann durch geeignete Diagnosemethoden, beispielsweise einem Computertomographen (CT) ermittelt werden. Die Lage des zu bestrahlenden Gebietes wird auf dem Patientenkörper angezeigt, indem die Projektion des zu bestrahlenden Gebietes auf der Haut eine Markierung erfährt. Mit Hilfe von im Bestrahlungsraum fest angeordneten Linienlasersystemen kann daher der Patient auf einer Liege in die präzise Lage gebracht werden. Mit Hilfe einer Sagittallinie kann der Patient zur Rotationsachse ausgerichtet werden.

Wie erwähnt, werden die Koordinaten des zu bestrahlenden Gebiets mit Hilfe eines Computertomographen ermittelt. Es ist daher notwendig, die ermittelten Koordinaten auf dem Patientenkörper so zu markieren, daß bei einer Ausrichtung des Patienten zum Bestrahlungsgerät das Zentrum des Tumors im Isozentrum des Bestrahlungsgerätes liegt.

Der Erfindung liegt daher die Aufgabe zugrunde, eine Vorrichtung zur Positionierung und Markierung eines Patienten mit Hilfe eines Computertomographen zu schaffen.

Diese Aufgabe wird durch die Merkmale des Patentanspruchs 1 gelöst.

Bei der erfindungsgemäßen Vorrichtung projiziert der rotierbare Linienlaser eine Linie auf den Körper des Patienten, der im Computertomographen (CT) diagnostiziert werden soll. In der Nullstellung ist diese Linie eine Sagittallinie, die zentral zur Gantry des CT in Richtung desselben auf den Patienten projiziert wird. Der Lagerkörper des Lasers kann zum Beispiel von einem Ring oder einer Scheibe realisiert werden, welche im rechten Winkel zur Achse des CT aufgestellt ist. Die Rotationsachse verläuft mithin durch die zentrale Achse des CT. Durch Drehung des Lagerkörpers kann die Laserlinie in beliebigem Drehwinkel auf dem Patienten abgebildet werden, und zwar in der Längsachse und immer auf die Rotationsachse (virtuelles Isozentrum) ausgerichtet. Die Drehbewegung erfolgt durch einen numerisch gesteuerten Antrieb, der von einer geeigneten Steuerelektronik gesteuert wird.

Durch die beschriebene Lagerung des Linienlasers ist die von ihm erzeugte Linie unabhängig von der winkelstellung im gleichen Abstand zum Patientenkörper und hat mithin die Projektion einer identischen Linie im Hinblick auf ihre Abmessungen und die Helligkeit zur Folge. Durch Eingabe von Steuerdaten zur Verstellung des Lagerkörpers kann die Linie nach Maßgabe der Koordinaten des zu bestrahlenden Gebietes, die durch den CT vorher ermittelt worden sind, verstellt werden. Dadurch ist es möglich, die Koordinaten von Tumoren auf den Körper äußerlich zu übertragen.

Nach einer Ausgestaltung der Erfindung ist der Lagerkörper mit Hilfe numerisch gesteuerter Antriebe seitlich und/oder in der Höhe verfahrbar. Dadurch kann die Rotationsachse des Lagerkörpers und damit des Linienlasers auf die zentrale Achse des zu bestrahlenden Gebietes ausgerichtet werden.

Eine weitere Ausgestaltung der Erfindung sieht vor, daß diametral am Lagerkörper zwei weitere Linienlaser angebracht sind, deren Lichtebenen seitlich auf beiden Seiten des Patientenkörpers stehen und senkrecht auf der Lichtebene des ersten Lasers und mit diesem ein virtuelles Isozentrum schneiden. Schließlich kann auch mindestens ein dritter Laser vorgesehen werden zur Erzeugung einer Transversallinie, wobei diese einen vorgegebenen Abstand zur Querachse des CT aufweist. Vorzugsweise ist der dritte Laser entlang einer Achse parallel zur zentralen Achse verstellbar gelagert und mit Hilfe eines geeigneten numerischen Antriebs verfahrbar.

Vor Beginn der Diagnose ist das beschriebene System in Nullstellung. Die Linienlaser auf dem Lagerkörper projizieren drei Linien, nämlich eine Sagittallinie und zwei laterale Linien, welche Linien sich im virtuellen Isozentrum schneiden. Der dritte Laser projiziert eine Transversallinie, die, wie erwähnt, einen vorgegebenen Abstand zur Querachse des CT aufweist. In der beschriebenen Nullstellung wird daher ein gleiches Koordinatenkreuz projiziert, wie bei dem eingangs beschriebenen fest installierten Lasersystem.

Nach diesem Liniensystem kann der Patient ausgerichtet werden, bevor er mit Hilfe einer verfahrbaren Liege der Gantry des CT zugeführt wird. Nach der Durchleuchtung (scanning) wird der Patient mit Hilfe einer geeigneten Antriebssteuerung, die von der Steuervorrichtung des CT gesteuert ist, in eine Position gefahren, in der das zu bestrahlende Gebiet in der Ebene der Transversallinie liegt. Damit liegt eine Koordinate für die vorzunehmende Markierung fest. Die übrigen Koordinaten, die sich ebenfalls aus dem Diagnoseergebnis des CT ergeben, werden in die Steuerelektronik für den Lagerkörper und damit die übrigen Laser eingegeben. Dies kann z.B. mit Hilfe einer Tastatur erfolgen, durch welche die Steuerdaten der Steuervorrichtung für die Antriebe vorgegeben werden, damit die Laser entsprechend verfahren werden. Die Verwendung einer manuell bedienbaren Tastatur hat den Vorteil, daß man von dem System eines speziellen CT unabhängig ist.

Durch Rotation des Lagerkörpers können die Laserlinien nacheinander in die gewünschten Positionen verfahren, so daß mit Hilfe der auf den Patientenkörper projizierten Laserlinien die im CT ermittelten Koordinaten des zu bestrahlenden Gebietes äußerlich auf den Patientenkörper übertragen und markiert werden können, um eine reproduzierbare Lagerung zum Isozentrum bei der Strahlentherapie zu erreichen.

Durch vertikales und seitliches Verfahren des rotierenden Systems kann, wie erwähnt, die Rotationsachse mit der zentralen Achse des zu bestrahlenden Gebietes zur Deckung gebracht werden.

Die Erfindung wird nachfolgend anhand von Zeichnungen näher erläutert.
- Fig. 1: zeigt Front- und Seitenansicht eines Lagerkörpers für Linienlaser für die erfindungsgemäße Vorrichtung.
- Fig. 2: zeigt die Seitenansicht der Vorrichtung nach Fig. 1 in bezug auf einen Computertomographen.
- Fig. 3: eine Eingabetastatur für die Vorrichtung nach Fig. 1.

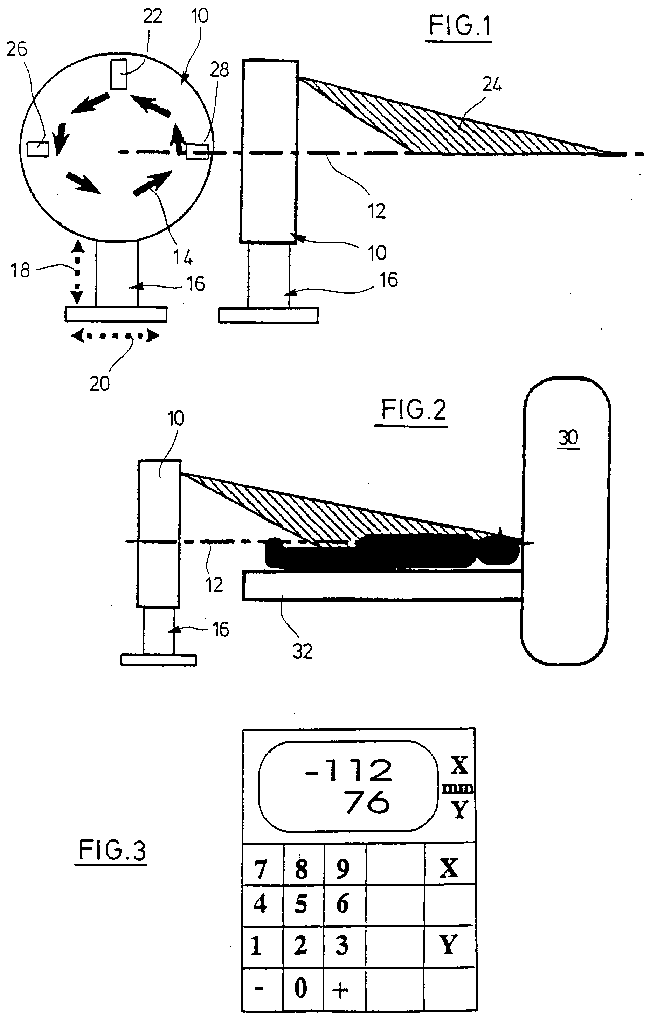

In Fig. 1 ist ein Drehkörper 10 zu erkennen, der um eine Achse 12 von einem nicht gezeigten numerisch gesteuerten Antrieb drehend antreibbar ist. Dies ist durch die Pfeile 14 angedeutet. Der Lagerkörper 10 ist auf einem Ständer 16 um die horizontale Achse drehbar gelagert, der eine Verstellung des Lagerkörpers 10 in vertikaler Richtung, wie durch Doppelpfeil 18 angedeutet und auch eine in horizontaler Richtung, wie durch Doppelpfeil 20 angedeutet, ermöglicht.

In Fig. 1 ist auf dem vertikalen Durchmesser liegend ein erster Linienlaser 22 angebracht, der in der gezeigten Nullposition eine Linie auf der Achse 12 erzeugt in einer Ebene 24 vertikal durch die Achse 12 gehend. Zwei weitere diametral gegenüberliegende Linienlaser 26, 28 erzeugen Linien in einer Horizontalebene senkrecht zur Ebene 24, wobei die Ebene 24 in der Achse 12 geschnitten wird.

In Fig. 2 ist zu erkennen, daß die Achse 12 zusammenfällt mit der Achse eines Computertomographen (CT), der mit 30 bezeichnet ist. Auf einer Liege 32 liegt ein zu untersuchender Patient. Die Liege ist horizontal verfahrbar in die Gantry des CT mit Hilfe eines numerisch gesteuerten Antriebs, der von Steuerdaten des CT gesteuert wird.

In einer Position der Liege 32 vor dem CT 30 kann der Patient nach den Linien der beschriebenen Laser 22, 26 und 28 ausgerichtet werden. Anschließend wird der Patient mit der Liege 32 in den Computertomographen hineingefahren. Nach dem sogenannten Scanning erfolgt mit Hilfe der Steuerung des CT ein Herausfahren der Liege 32 in einer Position dergestalt, daß der festgestellte Tumor in einer Transversalebene liegt, welche von ein oder zwei Linienlasern im Raum des CT erzeugt werden, wobei die Lichtebenen senkrecht auf den beschriebenen Lichtebenen der Laser 22, 26 und 28 stehen. Die übrigen X- und Y-Koordinaten, die durch das Scanning ermittelt worden sind, werden in ein Tableau gemäß Fig. 3 eingegeben. Eine nicht gezeigte Steuerung steuert dann die Antriebe für den Lagerkörper 10 an. Durch Rotation werden nacheinander die gewünschten Positionen für die Laser 22, 26 und 28 angefahren. Mit Hilfe der so auf den Körper projizierten Laserlinien können die im CT ermittelten Koordinaten des Tumors äußerlich auf den Körper des Patienten übertragen und markiert werden, um eine reproduzierbare Lagerung zum Isozentrum bei der Bestrahlung zu erreichen.

Durch ein vertikales und seitliches Verfahren des Lagerkörpers 10 kann seine Rotationsachse mit der zentralen Achse des Tumors in Deckung gebracht werden.

## Patentansprüche

1. Vorrichtung zur Positionierung und Markierung eines Patienten an Diagnosegeräten, z.B. vor und nach der Durchleuchtung in einem Computertomographen, mit Linienlasern, die im Raum in aufeinander senkrecht stehenden Ebenen Linien projizieren zur Ausrichtung auf ein zu bestrahlendes Gebiet des Patientenkörpers und Markierung desselben, dadurch gekennzeichnet, daß ein erster Linienlaser (22) auf einem Lagerkörper (10) angebracht ist, der um eine Achse (12) gelagert ist, die annähernd mit der zentralen Achse des Diagnosegerätes, z.B. Computertomographen (30) ausrichtbar ist, wobei der Lichtstrahl des außermittig angeordneten Linienlasers (22) in einer durch die Z-Achse hindurchgehenden Ebene liegt, die auf einer zwischen Diagnosegerät, z.B. Computertomograph (30) und Lagerkörper (10) angeordneten Liege (32) senkrecht steht, wenn der Lagerkörper (10) in Nullposition ist, daß der Lagerkörper (10) durch einen numerisch gesteuerten Antrieb antreibbar ist und eine Steuervorrichtung vorgesehen ist zur Verstellung des Lagerkörpers (10) nach Maßgabe von Steuerdaten, die aus den durch das Diagnosegerät, z.B. Computertomograph (30) ermittelten Koordinaten eines Tumors ermittelt werden.

2. Vorrichtung nach Anspruch 1, dadurch gekennzeichnet, daß die Steuervorrichtung ein Tasteneingabegerät (Fig. 3) aufweist zur Eingabe der durch das Diagnosegerät, z.B. Computertomograph (30) ermittelten Koordinaten bzw. den Steuerdaten.

3. Vorrichtung nach Anspruch 1, dadurch gekennzeichnet, daß die Steuervorrichtung von der Maschinensteuerung des Diagnosegerätes, z.B. Computertomograph, gesteuert ist.

4. Vorrichtung nach einem der Ansprüche 1 bis 3, dadurch gekennzeichnet, daß der Lagerkörper (10) mittels eines numerisch gesteuerten Antriebs in der Höhe verstellbar ist.

5. Vorrichtung nach einem der Ansprüche 1 bis 4, dadurch gekennzeichnet, daß diametral am Lagerkörper zwei weitere Linienlaser (26, 28) angebracht sind, deren Lichtebenen seitlich auf beiden Seiten des Patientenkörpers stehen und senkrecht auf der Lichtebene des ersten Lasers (22) und mit diesem ein virtuelles Isozentrum schneiden.

6. Vorrichtung nach einem der Ansprüche 1 bis 5, dadurch gekennzeichnet, daß der Lagerkörper (10) mittels eines numerisch gesteuerten Antriebs seitlich verstellbar ist.

7. Vorrichtung nach einem der Ansprüche 1 bis 6, dadurch gekennzeichnet, daß im Raum mindestens ein weiterer Linienlaser angeordnet ist zur Erzeugung einer Transversallinie.

8. Vorrichtung nach Anspruch 7, dadurch gekennzeichnet, daß der weitere Linienlaser entlang einer Achse parallel zur zentralen Achse des Diagnosegerätes, z.B. Computertomograph (30) mittels eines weiteren numerisch gesteuerten Antriebs verfahrbar ist.
